(19) Europäisches Patentamt
European Patent Office
Office européen des brevets

(11) Veröffentlichungsnummer: **0 540 894 A1**

(12) # EUROPÄISCHE PATENTANMELDUNG

(21) Anmeldenummer: **92117242.5**

(51) Int. Cl.5: **C07D 233/61**, C08G 59/02

(22) Anmeldetag: **09.10.92**

(30) Priorität: **07.11.91 DE 4136573**

(43) Veröffentlichungstag der Anmeldung:
**12.05.93 Patentblatt 93/19**

(84) Benannte Vertragsstaaten:
**CH DE ES FR GB LI**

(71) Anmelder: **Witco GmbH**
**Ernst-Schering-Strasse 14, Postfach 1620**
**W-4709 Bergkamen(DE)**

(72) Erfinder: **Burba, Christian, Dr. Dipl.-Chem.**
**Gerhardt-Hauptmann-Strasse 9**
**W-4715 Ascheberg-Herbern(DE)**
Erfinder: **Mrotzek, Werner, Dr. Dipl.-Ing.**
**Dresdener Strasse 24**
**W-4600 Dortmund 1(DE)**

(54) **Amid- und Carboxylgruppen enthaltende N-Aminoalkylimidazolverbindungen und deren Verwendung als Härtungsmittel für Epoxidharze.**

(57) Die Erfindung betrifft Amid- und Carboxylgruppen enthaltende N-Aminoalkylimidazolylderivate und ihre Verwendung als Härtungsmittel in faserverstärkten Kunststoffen auf Basis von Epoxidharzen.

EP 0 540 894 A1

Gegenstand der Erfindung sind neue Imidazolylderivate, ihre Verwendung als Härtungsmittel in Epoxidharzzusammensetzungen, sie enthaltende härtbare Epoxidharzzusammensetzungen, bestehend aus einem Epoxidharz und aus Amid- und Carboxylgruppen enthaltenden Verbindungen auf Basis von N-Aminoalkylimidazol-Säureanhydrid und gegebenenfalls Glycidylether-Reaktionsprodukten und gegebenenfalls üblichen Härtungs- und Lösungsmitteln zur Herstellung von Formkörpern.

Für die Herstellung von Verbundwerkstoffen werden heute im Prinzip zwei Verfahren angewandt.

Bei dem Naß-in-Naß-Verfahren werden die Verstärkungsmaterialien mit der härtbaren Mischung imprägniert und in der Hitze in einer Stufe bis zum duromeren Endzustand ausgehärtet (Einstufenverfahren).

Beim Zweistufenverfahren werden aus den Verstärkungsmaterialien und der härtbaren Mischung zunächst sogenannte Prepregs hergestellt, die dann in einem zeitlich getrennten zweiten Verfahrensschritt zu Fertigteilen weiterverarbeitet werden. Dabei ist verfahrenstechnisch noch einmal zwischen der Verwendung lösungsmittelhaltiger und lösungsmittelfreier Arbeitsweise zu unterscheiden.

Die Herstellung der Prepregs erfolgt normalerweise in einem kontinuierlichen Prozeß, in dem die Verstärkungsmaterialien entweder durch ein Imprägnierbad des einzusetzenden Harz-Härter-Gemisches geleitet werden oder das Imprägniermittel wird erst unmittelbar vor seinem Auftrag auf das Trägermaterial vermischt und mittels einer besonderen Vorrichtung aufgerakelt. Die Steuerung der auf eine bestimmte Trägermaterialbahn aufzubringenden Imprägniermittelmenge erfolgt dabei außer über die Viskosität des Imprägniermittels über nachgeschaltete Abquetschwalzen.

Bei lösungsmittelhaltigen Systemen wird nach dem Imprägnierprozeß durch Wärmezufuhr das in der Imprägnierlösung enthaltende Lösungsmittel verdampft und gleichzeitig das Harzsystem vom A- in den B-Zustand überführt. Aus den mit flüssigem bis stark klebrigem Imprägniermittel getränkten Verstärkungsmaterialien wird je nach Verfahrensbedingungen und verwendetem Harzsystem ein schwach klebriger bis fast trockener Prepreg. Bei diesem Verfahrensschritt ist es wichtig, daß einerseits das Lösungsmittel der Imprägniermischung vollständig entzogen wird, andererseits aber der für die Prepreg-Härtung im zweiten Verfahrensschritt notwendige latente Härter noch nicht anspringt, um ein ungewolltes Ausreagieren der imprägnierten Verstärkungsmaterialien zu verhindern.

Bei lösungsmittelfreien Systemen erfolgt abhängig von der chemischen Zusammensetzung des Harzsystems nach der Imprägnierung entweder ebenfalls eine kurze Wärmebehandlung des Materials, oder die Verstärkungsstoffe werden unmittelbar nach der Imprägnierung unter Verzicht auf eine gesonderte Wärmebehandlung beidseitig mit Trennfolien kaschiert und einer systemadäquaten Zwischenlagerung zugeführt. Im Verlaufe dieser Zwischenlagerung tritt entweder ein allmählicher Übergang des Harzsystems in den B-Zustand ein oder das Imprägniermittel wird unter weitgehendem Verzicht auf chemische Veränderungen allein durch physikalische Effekte auf den Trägermaterialien fixiert.

Die so erhaltenen Prepregs lassen sich als Rollen zwischenlagern und transportieren, ehe sie dem jeweiligen Anwendungsfall entsprechend zugeschnitten und in Bauteildicke übereinander geschichtet werden. Durch gleichzeitige Druck- und Temperatureinwirkung wird der Prepregstapel zu einem hochfesten Formteil ausgehärtet, wobei die noch niedermolekularen, fließfähigen Harze in den hochmolekularen C-Zustand des Duromers übergehen.

Während beim Einstufenverfahren lange offene Zeiten und kurze Aushärtungszeiten bei niedrigen Härtungstemperaturen gefordert werden, kommt beim Zweistufenverfahren als weiteres Kriterium noch eine möglichst lange Lagerstabilität der Prepregs hinzu. Dabei werden Lagerungstemperaturen unterhalb Raumtemperatur von der Praxis immer weniger akzeptiert.

Unabhängig von der Prepregherstellung soll ihre Aushärtung bei möglichst tiefen Temperaturen innerhalb praxisgerechter Zeit erfolgen, das Temperaturmaximum der exothermen Reaktion auch bei größeren Schichtdicken auf einem niedrigen Niveau verbleiben und das physikalische Eigenschaftsniveau der Endprodukte den Erfordernissen der Praxis angepaßt sein.

Zwar wurden bereits Härtungssysteme beschrieben, welche die Härtung deutlich unter 100 °C ermöglichen, jedoch besteht in der Praxis nach wie vor ein Bedarf an Niedrigsttemperaturhärtern mit gleichzeitig guter Lagerstabilität der reaktiven Mischung. Dieser Bedarf besteht insbesondere auf Gebieten, auf welchen großflächige Formteile erstellt werden müssen (Prepregs) und deren endgültige Aushärtung bei höheren Temperaturen besonderer apparativer Aufwendungen bedürfte.

Diese Forderungen hinsichtlich des Härtungsverhaltens und Eigenschaftsniveaus gelten in gleicher Weise auch für die im Naß-in-Naß-Verfahren zu verarbeitenden Epoxidharzsysteme.

Für gewisse Anwendungszwecke ist nur eine Anhärtung bis zur Formstabilität der Formkörper erforderlich bzw. sogar erwünscht, wobei nach einer eventuellen Zwischenlagerung die endgültige Aushärtung in einem nachgeschalteten Temperprozeß bei den erforderlichen Temperaturen erfolgt. Dabei ist es aber wichtig, daß bereits bei der Anhärtung die thermische Beständigkeit des Materials über die Härtungstemperatur steigt, da ansonsten die Entformung nur nach Absenkung der Formteiltemperatur möglich ist.

Das in härtbaren Mischungen auf Basis von Epoxidharzen seit langem als latenter Härter verwendete Dicyandiamid wird zur Erzielung der angestrebten Eigenschaften in der Regel mit Co-Härtungsmitteln und/oder Beschleunigern kombiniert. Aus der Literatur sind auf diesem Gebiet daher eine Vielzahl von Vorschlägen bekannt geworden.

Aufgabe der vorliegenden Erfindung war es, unter Vermeidung der Nachteile des Standes der Technik härtbare Mischungen auf Basis von Epoxidverbindungen und latenten und in den Epoxidharzen löslichen bzw. homogen verteilbaren Härtungsmitteln zu finden, welche bei niedrigen Temperaturen innerhalb praxisgerechter Zeit ohne hohe exotherme Temperaturspitzen zum formstabilen Zustand anhärten bzw. zum duromeren Endzustand aushärten, eine den Anforderungen der Praxis entsprechende thermische Beständigkeit besitzen, und welche in Prepregs bei Raumtemperatur über eine ausreichende Lagerstabilität verfügen.

Diese Aufgabe wird gelöst durch Verwendung neuer Amid- und Carboxylgruppen enthaltender Verbindungen auf Basis von N-Aminoalkylimidazol-Säureanhydrid und gegebenenfalls Glycidylether-Reaktionsprodukten.

Ein Gegenstand der Erfindung sind daher Verbindungen der allgemeinen Formel (I)

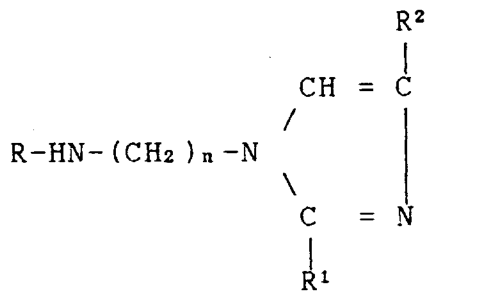

I

worin $R^1$ und $R^2$ unabhängig voneinander Wasserstoff, $CH_3$ oder $C_2H_5$ bedeuten, n = 2 oder 3 und R =

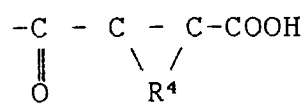

ist, worin $R^4$ ein Kohlenwasserstoffrest ist, welcher zusammen mit den mit ihm verbundenen C-Atomen einen gegebenenfalls substituierten aromatischen oder hydroaromatischen Sechsring bildet.

Ein weiterer Gegenstand der Erfindung sind Verbindungen der allgemeinen Formel (II)

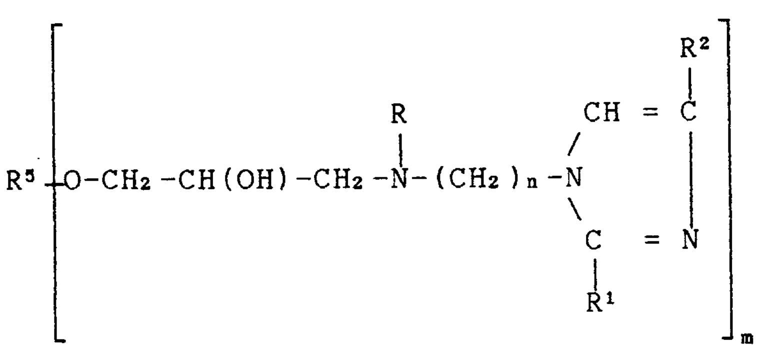

II

worin R die oben angegebene Bedeutung hat, $R^5$ der Rest der zur Herstellung der Glycidylether verwendeten Alkoholkomponente ist, $R^1$ und $R^2$ unabhängig voneinander Wasserstoff, $CH_3$, $C_2H_5$ bedeuten und $_n$ = 2 oder 3 ist und $_m$ gleich der Wertigkeit von R ist.

Ein weiterer Gegenstand der Erfindung sind Verbindungen, welche dadurch hergestellt sind, daß in erster Stufe Verbindungen gemäß Anspruch 2, dadurch herstellbar, daß in erster Stufe Ethergruppen

enthaltende Glycidylverbindungen an die primären Aminogruppen von N-Aminoalkylimidazolen, in einem Molverhältnis von primärer Aminogruppe zu Epoxidgruppe von ca. 1 : 1, addiert und in zweiter Stufe an die entstandenen sekundären Aminogruppen Carbonsäureanhydride, in einem Molverhältnis von sekundärer Aminogruppe zu Anhydridgruppe von ca. 1 : 1, addiert werden.

Ein weiterer Gegenstand der Erfindung ist dadurch gekennzeichnet, daß die Härtungsmittel gemäß den Ansprüchen 1 und 4 - 6 in Mengen von 2 bis 20 g, insbesondere 3 bis 10 g, und die Härtungsmittel gemäß den Ansprüchen 2 und 3 in Mengen von 5 bis 40, insbesondere 10 - 30, bezogen auf 100 g Epoxidharz, mitverwendet werden.

Ein weiterer Gegenstand der Erfindung ist dadurch gekennzeichnet, daß als Epoxidharze gemäß Anspruch 1 c) Glycidylether auf Basis von Bisphenol A, Bisphenol F und Novolaken mit Epoxidwerten von 0,18 - 0,6, insbesondere 0,39 - 0,55, mitverwendet werden. Handelsübliche halogenierte, insbesondere bromierte Epoxidharze mit ca. 18 Gew.-% Brom auf Basis der oben angegebenen Rohstoffe können ebenfalls verwendet werden.

Die erfindungsgemäßen Imidazolylverbindungen sind einmal herstellbar durch einfache Addition von Carbonsäureanhydriden an N-Aminoalkylimidazolen wie 1-(2-Aminoethyl)-imidazol und 1-(3-Aminopropyl)-imidazol nach an sich bekannten Methoden.

Die erfindungsgemäßen Imidazolylverbindungen sind zum anderen herstellbar durch Addition von Ethergruppen enthaltenden Glycidylethern an die primären Aminogruppen der N-Aminoalkylimidazolyl-Verbindungen, wobei das Verhältnis von Epoxidgruppen zu primären Aminogruppen ca. 1 : 1 beträgt, und man in zweiter Stufe an die entstandenen sekundären Aminogruppen Carbonsäureanhydride addiert, wobei pro Aminwasserstoff eine Anhydridgruppe eingesetzt wird. Die Additionsreaktion der ersten und zweiten Stufe werden nach den bekannten Verfahren durchgeführt.

Die erfindungsgemäß zur Herstellung der Imidazolylverbindungen mitverwendeten Ethergruppen enthaltenden Glycidylether sind Umsetzungsprodukte von ein- oder mehrwertigen Alkoholen und Epichlorhydrin zu den Chlorhydrinethern und anschließender Ringbildung mit Alkalihydroxiden. Diese Reaktionen erfolgen gemäß den zum bekannten Stand der Technik gehörenden Verfahren.

Als Alkohole können hier deren Ether- bzw. Estergruppen enthaltende höhermolekulare Folgeprodukte mit Molgewichten von ca. 500 - 1500 eingesetzt werden wie der Polyoxypropylendiglycidylether EUREPOX$^R$ RV-F der Schering AG, Bergkamen.

Die erfindungsgemäß mitverwendeten Imidazolylverbindungen sind Verbindungen der allgemeinen Formel III

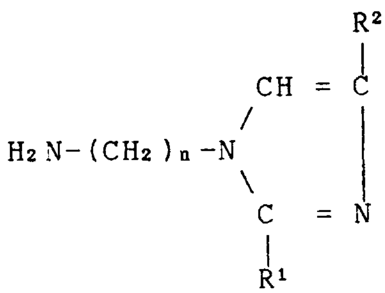

III

worin $R^1$ und $R^2$ unabhängig voneinander aliphatische oder aromatische Kohlenwasserstoff-Reste, vorzugsweise $CH_3$, $C_2H_5$ und insbesondere H bedeuten und n = 2 und insbesondere = 3 ist. Pro Epoxidgruppe der oben angeführten Glycidylverbindungen wird 1 mol der Imidazolylverbindung eingesetzt.

Als Carbonsäureanhydride sind die bekannten aliphatischen, cycloaliphatischen und aromatischen Anhydride mit mindestens einer Anhydridgruppe und gegebenenfalls einer oder mehrerer Carboxylgruppen pro Mol einsetzbar.

Erfindungsgemäß bevorzugt werden die Anhydride der Methyltetrahydrophthalsäure, Hexahydrophthalsäure, Pyromellitsäure, Trimellitsäure und insbesondere der Phthalsäure allein oder in Mischungen eingesetzt.

Pro sekundärer Aminogruppen der in erster Stufe hergestellten Additionsverbindungen wird eine Anhydridgruppe der Säureanhydridverbindungen eingesetzt.

Die Addition des Säureanhydrids an das N-Aminoalkylimidazol erfolgt nach an sich bekannten Verfahren, in welchen das Säureanhydrid in einem niedrigsiedenden Lösungsmittel und in einer Inertatmosphäre gelöst wird. Zu dieser Lösung wird das N-Aminoalkylimidazol, gegebenenfalls ebenfalls in einem

niedrigsiedenden Lösungsmittel gelöst, langsam zugetropft. Die Zutropfgeschwindigkeit wird dabei so geführt, daß die Reaktionstemperatur 50 °, vorzugsweise 40 °C, nicht überschreitet. Zur Vervollständigung der Reaktion wird 0,5 – 2 h bei dieser Temperatur nachgerührt und das Reaktionsprodukt durch die üblichen Verfahren vom Lösungsmittel getrennt.

Zur Herstellung der Verbindungen der allgemeinen Formel II wird die N-Aminoalkylverbindung gegebenenfalls unter Mitverwendung eines inerten Lösungsmittels in Stickstoffatmosphäre auf 40 – 100 °C, vorzugsweise 60 – 80 °C, aufgeheizt und der Glycidylether bzw. dessen Lösung in inerten Lösungsmitteln zugegeben. Zur Vervollständigung der Reaktion wird 0,5 – 2 h bei 60 – 80 °C weitergerührt, dann auf 100 – 130 °C aufgeheizt und das Säureanhydrid gegebenenfalls unter Mitverwendung von inerten Lösungsmitteln zugetropft. Nach Beendigung der Zugabe wird die Reaktion noch 0,5 – 2 h bei 100 – 120 °C weitergeführt und das Reaktionsprodukt durch die üblichen Verfahren vom Lösungsmittel getrennt.

Die erfindungsgemäß mitverwendeten Verbindungen gemäß den Ansprüchen wirken sowohl als Härtungsmittel als auch als Beschleuniger, so daß auf die mögliche Mitverwendung üblicher Beschleuniger und Härtungsmittel verzichtet werden kann. Andererseits können die erfindungsgemäßen Verbindungen mit Erfolg als Beschleuniger in handelsüblichen Härtungsmitteln, wie beispielsweise Dicyandiamid, eingesetzt werden.

Die Menge an Härtungsmitteln bzw. Härtungsbeschleunigern kann innerhalb weiter Grenzen variiert werden. Sie wird bestimmt durch den beabsichtigten Anwendungszweck und die gegebenenfalls dadurch vorgegebenen Härtungsbedingungen. Erfindungsgemäß werden Verbindungen der allgemeinen Formel I in Mengen im Bereich von 2 bis 20, vorzugsweise 3 bis 10 Gewichtsteilen, bezogen auf 100 Gewichtsteile Epoxidverbindung und die Verbindungen gemäß der allgemeinen Formel II in Mengen im Bereich von 5 – 40 g, insbesondere 10 – 30 g, bezogen auf 100 Gewichtsteile Epoxidverbindung, angewandt, wobei bei den halogenierten (bromierten) Epoxidharzen sich Mengen von 5 – 10 g/100 g Epoxidharz als vorteilhaft erwiesen haben.

Die zur Herstellung der härtbaren Mischung mitverwendeten Epoxidharze sind Glycidylester und -ether mit zwei oder mehr Epoxidgruppen pro Molekül wie vorzugsweise die Glycidylether auf Basis von ein- oder mehrwertigen Phenolen. Erfindungsgemäß bevorzugt werden Glycidylether von 2,2-Bis(4-hydroxyphenyl)-propan (Bisphenol A) mit Epoxidwerten von 0,2 – 0,6, insbesondere die bei Raumtemperatur halbfesten hochviskosen bis mittelviskosen Verbindungen mit Epoxidwerten um 0,39 bis 0,55. Daneben haben sich auch die Glycidylether auf Basis von Bisphenol F und die Novolake als vorteilhaft erwiesen.

Handelsübliche halogenierte, vorzugsweise bromierte Epoxidharze mit den bekannten Bromgehalten, vorzugsweise um ca. 18 Gew.-%, auf Basis der oben angeführten Harze können ebenfalls eingesetzt werden.

Zur Modifizierung der Eigenschaften des Endprodukts können neben anderen Epoxidharzen auch Modifizierungs- oder Hilfsstoffe mitverwendet werden wie Phenolharze, Melaminharze, Silikonharze.

Zur Einstellung der gewünschten Viskosität können unterschiedlich viskose Harze oder Verdünnungsmittel mitverwendet werden, aber auch die üblichen Lösungsmittel wie Dimethylformamid, Aceton, Methylethylketon, Methylglykol, Propylenglykolmonomethylether oder deren Mischungen eingesetzt werden.

Zur Herstellung der Prepregs werden organische und anorganische Fasern, Vliese und Gewebe, insbesondere aber Glas, mitverwendet.

Die Herstellung der lösungsmittelhaltigen Prepregs geschieht nach der an sich bekannte Methode, bei der die Trägermaterialien in einem Imprägnierbad mit der reaktiven Harzmischung getränkt und nach dem Abquetschen der überschüssigen Harzmenge kontinuierlich unter Energiezufuhr (meist Wärme) bei gleichzeitigem Entzug des Lösungsmittels vom A- in den B-Zustand überführt werden. Je nach gewünschter Prepreg-Konsistenz (klebrig bis fest) werden diese anschließend beidseitig mit einer Trennfolie versehen und für die Lagerung und den Transport aufgerollt. Die Weiterverarbeitung erfolgt durch Zuschneiden und Zusammenlegen der einzelnen Prepreg-Lagen zu einem Stapel, aus dem durch formgebende Maßnahmen unter gleichzeitiger Wärmezufuhr ein hochvernetztes Werkstück entsteht.

Die erfindungsgemäßen Härtungsmittel können außerdem mit Erfolg in lösungsmittelfreien Prepregs auf Basis von Epoxidharzen und gegebenenfalls üblichen Härtungsmitteln verwendet werden. Hierbei werden die Trägermaterialien bei gegebenenfalls erhöhter Temperatur nach an sich bekannten Verfahren mit den Bindemittelsystemen getränkt und systemadäquat gelagert, ehe sie wie die Lösungsmittel enthaltenden Systeme weiterverarbeitet werden.

Weitere Beispiele für lösungsmittelfreie Systeme sind unter anderem heißhärtende Einkomponentenklebstoffe z. B. für die Verklebung von Karosserieteilen in der Automobilindustrie und Epoxidharz-Beschichtungen, die entweder naß oder als Pulver aufgebracht werden.

Herstellung der erfindungsgemäßen Härtungsmittel

Beispiel 1

Umsetzungsprodukt von Phthalsäureanhydrid mit N-(3-Aminopropyl(imidazol

148 g Phthalsäureanhydrid werden unter Stickstoffatmosphäre in 550 ml Methylenchlorid gelöst. Zu dieser Lösung tropft man langsam 125 g N-(3-Aminopropyl)imidazol so zu, daß 38 °C nicht überschritten werden. Man rührt 1 h nach und läßt auf Raumtemperatur abkühlen. Das ausgefallene Reaktionsprodukt wird abbgesaugt und getrocknet.

| | |
|---|---|
| Schmelzpunkt: | 155 - 157 °C |
| Aminzahl: | 217/219. |

Beispiel 2

Umsetzungsprodukt von Phthalsäureanhydrid mit Addukt aus Polyoxypropylendiglycidylether und N-(3-Aminopropyl)imidazol

93,8 g N-(3-Aminopropyl)imidazol werden unter Stickstoffatmosphäre vorgelegt und bei 60 - 80 °C langsam mit 500 g Polyoxypropylendiglycidylether (Ep-Wert 0,2) versetzt. Man rührt 1 h bei 80 °C nach, erwärmt auf 110 - 120 °C und gibt 74,0 g Phthalsäureanhydrid portionsweise zu. Nachreaktion: 1 h bei 100 °C. Das Reaktionsprodukt hat folgende Kennzahlen:

| | |
|---|---|
| Viskosität/60 °C: | 12,5 Pa•s |
| Aminzahl: | 77. |

Beispiel 3

Umsetzungsprodukt von Methyltetrahydrophthalsäureanhydrid mit N-(3-Aminopropyl)-imidazol

83 g Methyltetrahydrophthalsäureanhydrid werden unter Stickstoff in 150 ml Methylenchlorid gelöst. Zu dieser Lösung tropft man langsam 62,5 g N-(3-Aminopropyl)-imidazol so zu, daß durch die exotherme Reaktion 38 °C nicht überschritten werden. Man rührt 1 h nach und zieht 20 g Lösungsmittel ab. Der bräunliche, hochviskose Rückstand hat eine Aminzahl von 203.

Ermittlung der Härtungsmitteleinflüsse in Verbindung mit bromierten Harzen

Beispiel 1

100 g eines in 25 g Methylethylketon (MEK) gelösten bromierten Epoxidharzes (Epoxidwert ca. 0,18, Bromgehalt ca. 18 %) werden mit 15 g einer 50 %igen Härterlösung aus dem erfindungsgemäßen Reaktionsprodukt, Ethanol und Wasser vermischt und zur Prepreg-Fertigung eingesetzt. Die Aufbereitung der Härterlösung erfolgt bei Raumtemperatur, wobei deren Konzentration grundsätzlich beliebig gewählt werden kann.

Zur Prepregherstellung im Labormaßstab wird zunächst ein ca. 0,1 $m^2$ großes Glasfilamentgewebe mit der Harz-Härter-Lösung getränkt. Daran anschließend erfolgt eine Wärmebehandlung des derart imprägnierten Verstärkungsmaterials von 2 min bei 120 °C in einem Umlufttrockenschrank. Während dieses Prepregvorgangs wird zum einen das Lösemittel aus dem Imprägniermittel entfernt, und zum zweiten werden die Reaktionskomponenten von dem A- in den B-Zustand übergeführt. Es resultieren nahezu trockene, flexible Prepregs, die nach der Abkühlung bei Raumtemperatur bis zur Weiterverarbeitung mehrere Wochen gelagert werden können.

Die Prepregweiterverarbeitung zu ca. 1 mm dicken Laminaten erfolgt in einer Stunde bei 150 °C mit einem Preßdruck von ca. 1 bar. Das derart ausgehärtete Endprodukt zeigt keinerlei Mängel bezüglich der

Haftung der einzelnen Prepreglagen.

Zur Bestimmung der thermischen Beständigkeit des Matrixmaterials werden anschließend aus den Laminaten 100 x 10 $mm^2$ große Probekörper entnommen und im Torsionsschwingversuch nach DIN 53455 untersucht. Die Ergebnisse dieser Prüfung sind für verschiedene Mischungsverhältnisse in Tabelle 1 wiedergegeben.

Die weiteren ebenfalls in Tabelle 1 aufgeführten TG-Werte der Beispiele 2 und 3 werden analog Beispiel 1 mit dem dort angeführten Epoxidharz ermittelt, wobei jedoch mit einer 50 %igen Härterlösung aus dem Reaktionsprodukt, Ethanol und Methylethylketon (MEK) gearbeitet wurde. Als Vergleichsgröße wird zusätzlich der TG eines derzeit häufig benutzten Matrixmaterials mit Dicyandiamid als Härter und N-Methylimidazol als Beschleuniger mit in die Tabelle aufgenommen (Mischungsverhältnis bromiertes Epoxidharz : Dicyandiamid : N-Methylimidazol = 100 : 5 : 0,8.

Tabelle 1

Thermische Beständigkeit der Laminate mit bromierten Harzen

| Härtungsmittel-Beispiel | Mischungsverhältnis Harz : Härter | TG in °C nach Prepreglagerung von 1 Tag | 4 Wochen | 12 Wochen |
|---|---|---|---|---|
| 1 | 100 : 7,5<br>100 : 15 | 129<br>125 | 120<br>119 | |
| 2 | 100 : 5<br>100 : 10 | 82<br>105 | 80<br>104 | <br>104 |
| 3 | 100 : 5<br>100 : 10 | 120<br>113 | 94 | |
| Vergleichsbeispiel | 100 : 5 : 0,8 | 118 | | |

<u>Ermittlung der Härtungsmitteleinflüsse in Verbindung mit BPA-Basisharzen</u>

<u>Beispiel 1</u>

100 g eines Epoxidharzes (Epoxid-Äquivalentgewicht ca. 190) werden mit 10 g des erfindungsgemäßen Reaktionsproduktes 1 vermischt und zur Prepreg-Herstellung eingesetzt.

Die Herstellung der Prepregs im Labormaßstab erfolgt durch Aufstreichen des Reaktionsgemisches auf ein ca. 0,1 $m^2$ großes Glasfilamentgewebe in Atlasbindung, welches nach der Imprägnierung beidseitig mit Trennfolien kaschiert und bei Raumtemperatur gelagert wird. Es resultieren stark klebende Prepregs, die ihre Konsistenz während der Lagerung nicht nennenswert verändern dürfen. Zur Weiterverarbeitung werden mehrere Lagen dieser Prepregs übereinander gelegt und bei einer Temperatur von 60 °C 12 h unter leichtem Druck (ca. 0,1 - 0,5 bar) verpreßt.

Zur Ermittlung des in Tabelle 2 angegebenen Zeitraumes gleichbleibender Prepregkonsistenz werden die Prepregs unter Normklima gelagert, und in Abständen von jeweils 24 h erfolgt eine Prepregverpressung entsprechend den vorstehend genannten Bedingungen. Als Zeitraum eines unveränderten Prepregzustandes ist der Tag angegeben, nach dem erstmalig Veränderungen im Harzfluß gemessen wurden.

Die Bestimmung der ebenfalls in Tabelle 2 enthaltenen thermischen Beständigkeit des Probenmaterials erfolgt an 100 x 10 $mm^2$ großen Probekörpern entsprechend DIN 53 455 im Torsionsschwingversuch, die aus den verpreßten Laminaten entnommen wurden.

Die weiteren in Tabelle 2 für die anderen Beispiele aufgeführten Tg-Werte und Zeiträume unveränderter Prepregkonsistenz werden analog Beispiel 1 ermittelt.

Tabelle 2

| Raumtemperatur-Lagerstabilitäten bei 60 °C gehärteter Prepregs | | | |
|---|---|---|---|
| Härtungsmittel-Beispiel | Mischungsverhältnis (Gew.-T.) Harz : Harter | TG in °C nach 16 h 60 °C Härtung | Zeitraum unveränderter Prepregkonsistenz |
| 1 | 100 : 10<br>100 : 20 | 78<br>82 | >5 Wochen<br>>5 Wochen |
| 2 | 100 : 20 | 74 | 4 Wochen |

Ermittlung der Härtungsmitteleinflüsse in Verbindung mit BPA-Basisharzen

Zur Ermittlung der Materialeigenschaften bei Verwendung der ausgewählten Härtungsmittel werden zur Vermeidung verfälschender Einflüsse von Verstärkungs- und Zusatzmaterialien die Mischungen, bestehend allein aus Epoxidharz und Härtungsmittel, ausgehärtet und abgeprüft.

In den in der Tabelle 3 aufgelisteten Beispielen wird als Epoxidharz ein Glycidylether auf Basis von Bisphenol A mit einem Epoxidwert von 0,53 verwendet.

Zur Herstellung der Prüfkörper werden jeweils 100 g des Epoxidharzes mit der in der Tabelle 3 genannten Menge Härtungsmittel bei Raumtemperatur vermischt und in einem Stahlwerkzeug zu 4 mm dicken ebenen Formteilen ausgehärtet. Aus diesen Formteilen werden dann durch Sägen bzw. Fräsen Probekörper entnommen, an denen unter Einhaltung der nachstehend genannten Prüfnormen die in Tabelle 3 aufgeführten Eigenschaftswerte ermittelt sind.

| | | Prüfkörperabmessungen |
|---|---|---|
| Biegefestigkeit | DIN 53 482 | 80 x 10 x 4 $mm^3$ |
| Durchbiegung | DIN 53 452 | 80 x 10 x 4 $mm^3$ |
| Schlagzähigkeit | DIN 53 453 | 50 x 6 x 4 $mm^3$ |
| Zugfestigkeit | DIN 53 455 | Schulterstab Nr. 3 |
| Dehnung | DIN 53 455 | Schulterstab Nr. 3 |
| Elastizitätsmodul | DIN 53 457 | Schulterstab Nr. 3 |
| Martenstemperatur | DIN 53 458 | 60 x 15 x 4 $mm^3$ |
| Heat Distortion Temperature (HDT) | DIN 53 461 | 120 x 10 x 4 $mm^3$ |
| Glasübergangstemperatur (TG) | DIN 53 445 | 80 x 10 x 1 $mm^3$ |

Tabelle 3

| Eigenschaften der unverstärkten Formstoffe | | | |
|---|---|---|---|
| Härtungsmittel | Beispiel 1 | Beispiel 2 | Beispiel 3 |
| Härtemenge (g)<br>Härtebedingungen | 10<br>2 h 120 °C | 20<br>2 h 120 °C | 10<br>2 h 120 °C |
| Biegefestigkeit (N/mm$^2$) | 55 | 75 | 95 |
| Durchbiegung(mm) | 5 | 11 | 12 |
| Schlagzähigkeit (kJ/m$^2$) | 3,5 | 13 | 20 |
| Zugfestigkeit (N/mm$^2$) | 42 | 32 | 50 |
| Dehnung (%) | 1,7 | 1,3 | 2,0 |
| Elastizitätsmodul (N/mm$^2$) | 2700 | 2600 | 2900 |
| Martenstemperatur (°C) | 95 | 70 | 109 |
| HDT (°C) | 128 | 115 | 132 |
| TG (°C) | 148 | 134 | 160 |

## Patentansprüche

**1.** Verbindungen der allgemeinen Formel (I)

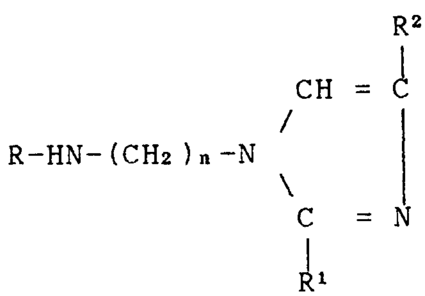

I

worin $R^1$ und $R^2$ unabhängig voneinander Wasserstoff, $CH_3$ oder $C_2H_5$ bedeuten, n = 2 oder 3 und R =

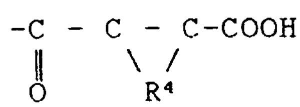

ist, worin $R^4$ ein Kohlenwasserstoffrest ist, welcher zusammen mit den mit ihm verbundenen C−Atomen einen gegebenenfalls substituierten aromatischen oder hydroaromatischen Sechsring bildet.

**2.** Verbindungen der allgemeinen Formel (II)

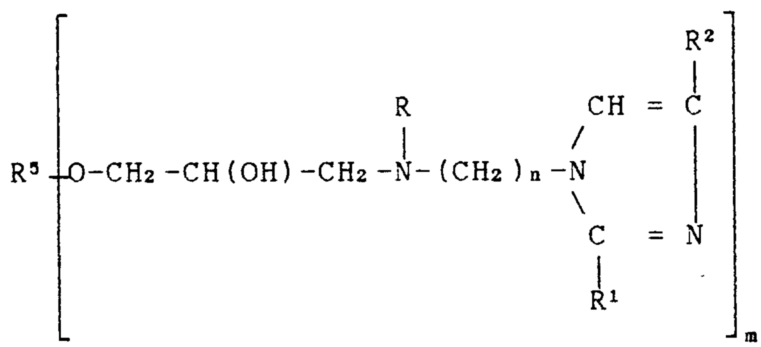

II

worin R die oben angegebene Bedeutung hat, $R^5$ der Rest der zur Herstellung der Glycidylether verwendeten Alkoholkomponente ist, $R^1$ und $R^2$ unabhängig voneinander Wasserstoff, $CH_3$, $C_2H_5$ bedeuten und $_n$ = 2 oder 3 ist und $_m$ gleich der Wertigkeit von R ist.

**3.** Verbindungen gemäß Anspruch 2, dadurch herstellbar, daß in erster Stufe Ethergruppen enthaltende Glycidylverbindungen an die primären Aminogruppen von N-Aminoalkylimidazolen, in einem Molverhältnis von primärer Aminogruppe zu Epoxidgruppe von ca. 1 : 1, addiert und in zweiter Stufe an die entstandenen sekundären Aminogruppen Carbonsäureanhydride, in einem Molverhältnis von sekundärer Aminogruppe zu Anhydridgruppe von ca. 1 : 1, addiert werden.

**4.** Verbindungen gemäß den Ansprüchen 1 und 3, dadurch gekennzeichnet, daß als Säureanhydrid Phthalsäureanhydrid mitverwendet wird.

**5.** Verbindungen gemäß den Ansprüchen 1 bis 4, dadurch gekennzeichnet, daß als Imidazolverbindung 1-(3-Aminopropyl)-imidazol mitverwendet wird.

**6.** Verbindungen gemäß den Ansprüchen 2 bis 5, dadurch gekennzeichnet, daß als Glycidylverbindung ein Polyoxypropylendiglycidylether mit einem Epoxidwert von 0,18 - 0,22 mitverwendet wird.

**7.** Verwendung der Verbindungen gemäß den Ansprüchen 1 bis 6 als Härtungsmittel für Epoxidharze, wobei die Verbindungen gemäß Anspruch 1 in Mengen von 2 - 20 g, die Verbindungen gemäß den Ansprüchen 2 und 3 in Mengen von 5 - 40 g, bezogen auf 100 g Epoxidharz, mitverwendet werden.

**8.** Härtbare Epoxidharzzusammensetzung, enthaltend
a) ein Epoxidharz mit durchschnittlich mehr als einer Epoxidgruppe pro Molekül und
b) mindestens eine der Verbindungen gemäß den Ansprüchen 1 bis 6

**9.** Härtbare Epoxidharzzusammensetzungen, worin die Verstärkungs- und Einlagematerialien bei Raumtemperatur mit dem Bindemittel, bestehend aus
a) einem Epoxidharz mit im Durchschnitt mehr als einer Epoxidgruppe pro Molekül und
b) mindestens einer Verbindung gemäß den Ansprüchen 1 bis 6 und gegebenenfalls
c) Lösungsmitteln, Füllstoffen, Pigmenten, Hilfsstoffen
getränkt und gegebenenfalls bei erhöhter Temperatur in den halbfesten aber noch schmelzbaren Zustand (B-Zustand) überführt werden.

**10.** Härtbare Epoxidharze gemäß den Ansprüchen 8 und 9, dadurch gekennzeichnet, daß als Epoxidharze Glycidylether auf Basis von Bisphenol A, Bisphenol F und Novolaken mit Epoxidwerten von 0,18 - 0,6 mitverwendet werden.

**11.** Epoxidharzformkörper, dadurch gekennzeichnet, daß in erster Stufe die Verstärkungs- und Einlagematerialien bei Raumtemperatur mit dem Bindemittel, bestehend aus
a) einem Epoxidharz mit im Durchschnitt mehr als einer Epoxidgruppe pro Molekül und
b) mindestens einer der Verbindungen gemäß den Ansprüchen 1 bis 6 und gegebenenfalls
c) Lösungsmitteln, Füllstoffen, Pigmenten, Hilfsstoffen
überführt werden und in zweiter Stufe die Laminate oder Prepregs unter Formgebung oder zwischen zu

verklebende Substrate gebracht, unter Anwendung von Druck bei erhöhter Temperatur ausgehärtet werden.

**12.** Verfahren zur Herstellung von faserverstärkten Trägermaterialien für den Elektrosektor durch Überführung von mit Bindemittel auf Basis von Epoxidharzen und aminischen Härtungsmitteln imprägnierten Verstärkungsmaterialien in erster Stufe durch Anwendung von Temperatur und gegebenenfalls von Druck in den B–Zustand überführt und in zweiter Stufe bei erhöhter Temperatur ausgehärtet werden, dadurch gekennzeichnet, daß als Epoxidharze
a) bromierte Epoxidharze mit im Durchschnitt mehr als einer Epoxidgruppe pro Molekül
und als aminische Härtungsmittel
b) mindestens eine der Verbindungen gemäß den Ansprüchen 1 bis 6 verwendet werden.

**13.** Verfahren zur Herstellung von faserverstärkten Trägermaterialien gemäß Anspruch 9, dadurch gekennzeichnet, daß als Epoxidharze bromierte Epoxidharze mit ca. 18 Gew.–% Brom mitverwendet werden.

Europäisches Patentamt

# EUROPÄISCHER RECHERCHENBERICHT

Nummer der Anmeldung

EP 92 11 7242

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 450 322 (SCHERING AG)<br>* Ansprüche *<br>--- | 1-13 | C07D233/61<br>C08G59/02 |
| A | EP-A-0 417 498 (SCHERING AG)<br>* Ansprüche *<br>--- | 1-13 | |
| P,A | EP-A-0 457 046 (SCHERING AG)<br>* Ansprüche *<br>----- | 1-13 | |

| RECHERCHIERTE SACHGEBIETE (Int. Cl.5 ) |
|---|
| C07D<br>C08G |

Der vorliegende Recherchenbericht wurde für alle Patentansprüche erstellt

| Recherchenort | Abschlußdatum der Recherche | Prüfer |
|---|---|---|
| DEN HAAG | 12 MAERZ 1993 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)